**Europäisches Patentamt**

**European Patent Office**

**Office européen des brevets**

(19)

(11) Veröffentlichungsnummer: **0 101 963**
**A1**

# (12) EUROPÄISCHE PATENTANMELDUNG

(21) Anmeldenummer: **83107539.5**

(22) Anmeldetag: **30.07.83**

(51) Int. Cl.³: **A 61 K 9/08**
**A 61 K 31/52**

(30) Priorität: **04.08.82 FR 8213610**

(43) Veröffentlichungstag der Anmeldung:
**07.03.84 Patentblatt 84/10**

(84) Benannte Vertragsstaaten:
**AT BE CH DE GB IT LI LU NL SE**

(71) Anmelder: **LABORATOIRES HOECHST S.A.**
**Tour Roussel Nobel 3, Avenue du Général de Gaulle**
**F-92800 Puteaux(FR)**

(72) Erfinder: **Benyaya, Joseph**
**39 la Côte Douce**
**F-61300 L'Aigle(FR)**

(74) Vertreter: **Meyer-Dulheuer, Karl-Hermann, Dr. et al,**
**HOECHST Aktiengesellschaft Zentrale Patentabteilung**
**Postfach 80 03 20**
**D-6230 Frankfurt/Main 80(DE)**

(54) Neue, oral verabreichbare galenische Formen von löslichen Dimethylxanthinderivaten.

(57) Die vorliegende Erfindung betrifft neue galenische, oral verabreichbare Formen von löslichen Dimethylxanthinderivaten.

Diese galenische Form ist dadurch gekennzeichnet, daß sie in Form einer wäßrigen Lösung vorliegt, die ein Dimethylxanthinderivat in Kombination mit Sorbit und Carboxymethylcellulose enthält.

EP 0 101 963 A1

LABORATOIRES HOECHST S.A.    HOE 82/S 036    Dr.D /St

Neue, oral verabreichbare galenische Formen von löslichen
Dimethylxanthinderivaten

Die vorliegende Erfindung betrifft neue, oral verabreichbare galenische Formen von löslichen Dimethylxanthinderivaten.

Dimethylxanthinderivate, insbesondere das Pentoxifyllin,
sind für ihre gefäßerweiternde Wirkung durch direktes Einwirken auf die glatten Gefäßmuskelfasern und insbesondere
durch ihre Wirkung auf die roten Blutkörperchen bekannt:
in der Tat wirken sie einer Verringerung der Erythrocytenverformbarkeit entgegen.

Weiterhin finden diese Derivate breite Anwendung in der
Medizin, insbesondere bei zerebralen ischämischen Vorfällen, bei peripherer Kreislaufinsuffizienz und bei
allen Arterienerkrankungen, insbesondere der unteren Gliedmaßen. Leider haben diese Derivate unerwünschte Nebenwirkungen im Verdauungstrakt, wie Brechreiz, Erbrechen
und insbesondere Sodbrennen, sowie Magenschmerzen. Aufgrund
dieser Unverträglichkeit sind der Anwendung dieser ausgezeichneten Arzneimittel Grenzen gesetzt. Deshalb hat man
seit einigen Jahren versucht, hier durch die Entwicklung
einer neuen, festen Arzneimittelform mit Überzug, die
einen Schleimbildner, wie z.B. Hydroxyethylcellulose enthält, häufig in Verbindung mit Polyvinylpyrrolidon (siehe
insbesondere das französische Patent 2 310 764) Abhilfe
zu schaffen. Das so erhaltene Medikament ist insbesondere
gegenüber der bisherigen Arzneimittelform insofern verbessert worden, als Magenschmerzen nach seiner Verabreichung praktisch verschwunden sind, und bei Versuchen, sowohl in vitro als auch in vivo zeigte sich, daß der Wirkstoff
in konstanten Mengen und über einen langen Zeitraum im
Magen-Darm-Trakt resorbiert wird.

Allerdings besteht nach wie vor ein großer Nachteil, der in der festen Form des Medikaments selber zu sehen ist, einer Form, die wesentlich weniger anpassungsfähig und wesentlich schlechter in der Handhabung ist, als eine flüssige Arzneimittelform.

Aufgabe der vorliegenden Erfindung war es daher, neue oral verabreichbare galenische Arzneimittelformen ohne jegliche Nebenwirkungen wie Magenschmerzen, selbst bei Langzeitbehandlung, zur Verfügung zu stellen. Aufgabe der Erfindung war es, eine flüssige, leicht zu handhabende Arzneimittelform, die für den Magen-Darm-Trakt absolut verträglich ist, unabhängig von der Behandlungsdauer und von der verabreichten Menge, zur Verfügung zu stellen, eine Arzneimittelform, die bislang nicht existierte.

Die vorliegende Erfindung betrifft eine neue, oral verabreichbare galenische Form von Dimethylxanthinderivaten, dadurch gekennzeichnet, daß sie in Form einer wäßrigen Lösung, die ein Dimethylxanthinderivat in Kombination mit Sorbit und Carboxymethylcellulose enthält, vorliegt.

In einer vorteilhaften Ausführungsform des Erfindungsgegenstandes enthält die Kombination pro Teil Dimethylxanthinderivat 4 - 8 Teile Sorbit und 0,5 - 1 Teil Carboxymethylcellulose.

In einer anderen vorteilhaften Ausführungsform des Erfindungsgegenstandes enthält die Lösung außerdem Geschmacksstoffe und/oder Aromastoffe und/oder Konservierungsmittel.

Erfindungsgemäß beträgt die Konzentration des Dimethylxanthinderivates in der wäßrigen Lösung 2 - 6 % (Gewicht/Volumen).

In einer besonders vorteilhaften Ausführungsform des Er-

0101963

findungsgegenstandes verwendet man als Dimethylxanthinderivat das Pentoxifyllin.

Weiterhin hat die Anmelderin gefunden, daß sich das erfindungsgemäße Arzneimittel besonders gut zur wirksamen
Bekämpfung einer so gefürchteten Krankheit wie der Sichelzellenanämie eignet.

Zusätzlich zu dem, was oben beschrieben wurde, betrifft
die vorliegende Erfindung noch weitere Aspekte, die aus
nachfolgender Beschreibung ersichtlich sind:

Die Erfindung betrifft insbesondere die neue erfindungsgemäße galenische Form, Verfahren und Mittel zu ihrer
Herstellung sowie ihre Anwendung, insbesondere zur Behandlung der Sichelzellenanämie.

Die Erfindung wird anhand der folgenden Beschreibung,
die sich auf ein Herstellungsbeispiel der erfindungsgemäßen Arzneimittelform sowie auf einen pharmakologischen
Bericht bezieht, besser verständlich.

Dieses Beispiel und der Bericht dienen selbstverständlich nur zur Erläuterung des Erfindungsgegenstandes und
stellen keinerlei Einengung dar.

Herstellungsbeispiel:
Man löst 1,0937 g Methylparaben und 0,468 g Propylparaben
in 800 ml auf 80°C erhitztem destilliertem Wasser. Dann
läßt man auf 40°C abkühlen und gibt 50 g Pentoxifyllin
sowie 3 g Natriumsaccharinat zu. Unter Rühren gibt man
dann 312,5 g einer 70 %igen Sorbit (USP XX) Lösung zu.
Man kühlt auf 30°C ab und rührt anschließend 37,5 g
Carboxymethylcellulose in fein verteilter Form unter.
Dann gibt man Aromastoffe zu und füllt mit destilliertem
Wasser auf 1250 ml auf.

Magenverträglichkeitstest:

Diese Versuche (Blindversuche) wurden an 18 Testpersonen durchgeführt, die entweder

- das Placebo oder
- die obige erfindungsgemäße Lösung erhielten.

Dabei wurden folgende Ergebnisse erzielt:

Placebo: Keine Magenschmerzen

Erfindungsgemäße Lösung: Keine Magenschmerzen.

Wirkung bei der Sichelzellenanämie:

Aus ersten Versuchsergebnissen, die sich auf 10 Kinder beziehen, ist eine bedeutende Abnahme der Anzahl von Krisen vom 4. Behandlungsmonat an erkennbar: im Durchschnitt 0,075 Krise pro Kind und pro Monat über einen Zeitraum von 6 Monaten, gegenüber 0.32 Krise pro Kind und pro Monat vorher.

Die verabreichten Tagesdosen waren 1 g im Falle des Placebo und 20 ml im Falle der erfindungsgemäßen Arzneimittelform.

Aus diesen Ergebnissen läßt sich eindeutig ablesen, daß die Anzahl der Krisen bei den mit der erfindungsgemäßen Arzneimittelform behandelten Patienten deutlich abnahm.

Die erfindungsgemäße Arzneimittelform ist absolut stabil (ohne daß die Wirkung nach 3-monatiger Lagerung bei 40°C nachläßt) und sie eignet sich insbesondere für die Kindertherapie (für alle Indikationen des Pentoxifyllins), da sie absolut verträglich und ideal in der Anwendung ist.

Beispielsweise kann man im Falle einer Tagesdosis von 10 mg/kg eine Formulierung mit 400 mg Dimethylxanthinderivat pro 10 ml Lösung verwenden, die zweimal täglich mit einem 5 ml fassenden Löffel mit Markierung für eine

0101963

halbe Füllung (2,5 ml) verabreicht wird:
bei einem Kind von 30 kg Körpergewicht: 1,5 Löffel pro
Tag und bei einem Kind von 50 kg Körpergewicht: 2 Löffel
pro Tag.

Wie aus den obigen Erläuterungen ersichtlich wird, beschränkt sich die Erfindung keineswegs auf die obige
Arzneiform; sowie die obigen Herstellungs- und Anwendungsarten, sondern erstreckt sich auch auf alle für
den Fachmann möglichen Varianten, ohne daß der Erfindungsrahmen gesprengt und die Reichweite der vorliegenden
Erfindung überschritten würden.

PATENTANSPRÜCHE:

1. Neue, galenische, oral verabreichbare Form von Dimethylxanthinderivaten, dadurch gekennzeichnet, daß sie in Form einer wäßrigen Lösung vorliegt, die ein Dimethylxanthinderivat in Kombination mit Sorbit und Carboxymethylcellulose enthält.

2. Arzneimittelform nach Anspruch 1, dadurch gekennzeichnet, daß obige Kombination pro Teil Dimethylxanthinderivat 4 - 8 Teile Sorbit und 0,5 - 1 Teil Carboxymethylcellulose enthält.

3. Arzneimittelform nach einem der Ansprüche 1 und 2, dadurch gekennzeichnet, daß die Lösung noch Süßstoffe und/oder Aromastoffe und/oder Konservierungsmittel enthält.

4. Arzneimittelform nach einem der Ansprüche 1 bis 3, dadurch gekennzeichnet, daß die Konzentration des Dimethylxanthinderivats in der wäßrigen Lösung 2 - 6 % (Gew./Vol.) beträgt.

5. Arzneimittelform nach einem der Ansprüche 1 bis 4, dadurch gekennzeichnet, daß das Dimethylxanthinderivat Pentoxifyllin ist.

6. Anwendung der Arzneimittelform nach einem der Ansprüche 1 bis 5 für die Behandlung der Sichelzellenanämie.

## EUROPÄISCHER RECHERCHENBERICHT

Europäisches Patentamt

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. ³) |
|---|---|---|---|
| A | GB-A- 867 803 (R.J. STRASENBURGH CO.) * Ansprüche 1,5; Seite 1, Zeilen 42-65; Beispiel 1; Seite 3, Zeilen 69-70 * | 1,3 | A 61 K 9/08 A 61 K 31/52 |
| | --- | | |
| A | CHEMICAL ABSTRACTS, Band 86, Nr. 8, 21. Februar 1977, Seite 237, Nr. 47273g, Columbus, Ohio, US NADIA M. MURSI u.a.: "Rheological studies of some natural and synthetic polymers upon mixing with certain polyhydroxy compounds" & BULL. FAC. PHARM., CAIRO UNIV. 1974, 13(1), 94-104 * Zusammenfassung * | 1 | |
| | --- | | |
| A | FR-A-2 421 614 (ISTITUTO BIOLOGICO CHEMIOTERAPICO) * Ansprüche 1,3; Beispiel b; Seiten 8-9 * | 1,3,4 | RECHERCHIERTE SACHGEBIETE (Int. Cl. ³) |
| | --- | | A 61 K 9/00 A 61 K 31/00 |
| A | CHEMICAL ABSTRACTS, Band 80, Nr. 3, 21. Januar 1974, Seite 336, Nr. 13810r, Columbus, Ohio, US ROSE M. PANGBORN u.a.: "Effect of hydrocolloids on oral viscosity and basic taste intensities" & J. TEXTURE STUD. 1973, 4(2), 224-41 * Zusammenfassung * | 1 | A 61 K 47/00 |
| | ----- | | |

Der vorliegende Recherchenbericht wurde für alle Patentansprüche erstellt.

| Recherchenort | Abschlußdatum der Recherche | Prüfer |
|---|---|---|
| DEN HAAG | 21-11-1983 | WILLEKENS G.E.J. |